# EUROPEAN PATENT SPECIFICATION

(11) **EP 1 082 448 B1**
(45) Date of publication and mention of the grant of the patent: **09.11.2005**
(21) Application number: 99919468.1
(22) Date of filing: 21.05.1999
(51) Int. Cl.: C12P 7/06, C12C 11/00, C12G 1/00, C12G 3/00, C12G 3/02, C12N 1/16, C12N 1/18, C12C 11/02

(54) **IMPROVED PROCESS FOR ALCOHOLIC FERMENTATION**
VERBESSERTES VERAHREN ZUR FERMENTATIVEN HERSTELLUNG VON ALKOHOL
PROCEDE AMELIORE DE FERMENTATION ALCOOLIQUE

(30) Priority: 23.05.1998 GB 9811103
(43) Date of publication of application: 14.03.2001
(73) Proprietor: Danstar Ferment AG, 6301 Zug (CH)
(72) Inventor: MCLAREN, James, F-32700 Lectoure (FR)
(74) Representative: Peaucelle, Chantal
(86) International application number: PCT/IB1999/000924
(87) International publication number: WO 1999/061646

(56) References cited:
- WO-A-93/01298
- CHEMICAL ABSTRACTS, vol. 128, no. 2, 12 January 1998 (1998-01-12) Columbus, Ohio, US; abstract no. 12760, BAKOYIANIS, V. ET AL: "Comparative Study of Kissiris,. gamma.- Alumina, and Calcium Alginate as Supports of Cells for Batch and Continuous Wine-Makin at Low Temperatures" XP002111347 & J. AGRIC. FOOD CHEM. (1997), 45(12), 4884-4888 ,
- CHEMICAL ABSTRACTS, vol. 116, no. 9, 2 March 1992 (1992-03-02) Columbus, Ohio, US; abstract no. 82160, ICONOMOU, L. ET AL: "The promotion of molasses alcoholic fermentation using Saccharomyces cerevisiae in the presence of. gamma.- alumina" XP002111348 & APPL. BIOCHEM. BIOTECHNOL. (1991), 31(1), 83-96 ,
- CHEMICAL ABSTRACTS, vol. 96, no. 19, 10 May 1982 (1982-05-10) Columbus, Ohio, US; abstract no. 161190, KAKO HONSHA K. K., JAPAN: "Mineral - rich health food materials" XP002111349 & JP 57 018969 A (KAKO HONSHA K. K., JAPAN) 1980

## Description

The present invention generally relates to an improved process for alcoholic fermentation which comprises the use of a mineral-rich or mineral-enriched yeast as a nutrient in said fermentation process, and to the use of such a yeast as a nutrient in an alcoholic fermentation process.

There are a number of minerals that are required in trace amounts for efficient alcoholic fermentation. These in particularly include metals capable of alterating the fermentation metabolism, such as divalent metals *e.g.* manganese, magnesium and zinc. There has been an increasing awareness of the importance of such trace minerals in alcoholic fermentations, particularly with respect to beer.

The zinc concentration of a wort is of particular importance from two perspectives. Firstly, if limiting, it can lead to sub-optimal, even incomplete fermentations, problems with head retention and yeast flocculance. Secondly, adequate levels of zinc can aid in the optimisation of alcoholic fermentations, vis a vis ethanol production and fermentable sugars uptake. This second perspective has a greater importance during fermentations when the yeast is subject to greater stresses. Moreover, traditionally, breweries recycle their yeast from one fermentation to another. Repitching yeast from one fermentation where the zinc is limiting into another beer wort, which is also deficient, would exacerbate fermentation problems.

Other minerals have been shown to be of importance in the course of an alcoholic fermentation. Manganese is thus known to be implicated as a substitute zinc metabolism, and could possibly mitigate some toxic effects associated with high concentrations of zinc. Another example is magnesium which is reported to be important for alcohol efficiency in fermentations. This is particularly a problem for the fermentation of certain substrates where there is an excess of calcium ions present. Calcium is indeed known to be antagonistic to magnesium metabolism and, for example, in beer, calcium is deliberately added in order to control the pH (acidity) and activate some of the enzymes of the malted barley.

For most alcoholic fermentations, there is thus a perceived natural mineral deficit in the substrate, and minerals, in the form of mineral salts such as zinc/manganese/magnesium chloride or sulphate, are generally added directly into the substrate, e.g. into the wort at the boiling stage for beer production. The use of such mineral salts, whilst relatively effective, conflicts with the desire by some industrialists to produce additive free alcohols.

Alternatives to the addition of mineral salts have thus been proposed in the past decades. These include pre-loading the fermentation yeast with a metal in such a way that the metal is hardly released from the cell body of the fermentation yeast during the fermentation process (JP 63287474), or using ash trub or acid extracts of spent grains or hop trub (US 4,840,802) so as to make use of the trace elements they contain. But all these alternatives are, in terms of quantity and quality of alcoholic fermentation production, at the best only substantially equivalent to the initial solution of directly adding mineral salts. Their industrial application is thus quite restricted, and some of them even show problems of off-odours associated with the process (*e.g.* acid extract use). None of the prior art techniques thus provides with fully satisfactory results.

It is an object of the present invention to provide with an improved process for alcoholic fermentation which, further to not showing the drawbacks of prior art techniques, is quantitatively, and also qualitatively more efficient than the solution of directly adding mineral salts or any other alternative solution. The process and use according to the invention allow an enhanced fennentation yeast growth, and accelerated fermentation. It also shows many advantages : it is very easy to handle, it applies to any alcoholic fermentation process, and is economically very beneficial. The process of the invention comprises the use of at least one mineral-rich or mineral-enriched yeast not as fermentation micro-organism, but as a nutrient source : the present invention indeed shows that, contrary to the received wisdom in the brewing industry, another micro-organism can be efficiently added to a fermentation process without leading to microbial instability, and that it is moreover able to provide the fermentation micro-organism with nutrients, and particularly with minerals such as zinc, magnesium, manganese in a very efficient way. As will be further described and illustrated below, this efficiency as a mineral source not only lies into an efficient mineral flux from the yeast(s) used as a nutrient source towards the fermentation micro-organism : the process and use according to the invention are indeed more efficient than the direct addition into the substrate of an equivalent quantity of mineral salt, and is even more efficient than the separate addition of both mineral salt on one hand, and a dead yeast on the other hand (see examples). That is to say, the process according to the invention shows synergetic effects in terms of mineral nutrition. These synergetic effects may at least partially lie in an increased mineral bioavailability favorable to the fermentation micro-organism.

The term "fermentation process" is herein meant as including the whole production process, and is no way limited to the precise biological step of fermentation. It e.g. also includes the fermentation yeast propagation step and the process of production of the substrate. The term "nutrient" herein comprises any element which can be considered of nutritive value to the fermentation micro-organsm, and thus also comprises micro- or trace nutrients. It has to be also pointed out that the word "yeast" is herein meant as a yeast cell which can be living or dead, and which still comprises at least one structure corresponding to an insoluble cell structure.

A preferred yeast for use according to the invention is a mineral-enriched yeast. Said at least one mineral-rich or mineral-enriched yeast is advantageously chosen among the food grade yeast genera. Examples of appropriate yeasts include the *Saccharomyces* genera (*e.g. Saccharomyces cerevisiae*) and the *Kluyveromyces* genera.

In an embodiment of the present invention, said at least one mineral-rich or mineral-enriched yeast is, before use, such as obtained by adding about 1,000 to about 200,000 ppm (relative to the weight of the yeast, as measured on a dry weight basis) of a salt of said mineral to a live culture of yeast at a temperature of about 4-40°C, preferably about 25-32°C, at a pH of between about 3.5 to about 7.0, preferably about 4.6 to 6.6, for a period of about 1-20 or 1-24 hours, preferably 2-16 hours so as to allow said yeast to incorporate, absorb and/or adsorb the mineral(s). Any salt, e.g. acetate, caprylate, carbonate, chloride, chromate, gluconate, iodate, lactate, oleate, oxide, perchlorate, peroxide, phosphate, salicylate, sulphate, sulphide, tartarate or valerate is appropriate. Comparative assays can be performed by the person skilled in the art to determine the most efficient mineral source. Said mineral incorporation can correspond to an absorption and/or an adsorption. When incorporated, said mineral may remain as a mineral and/or be transformed into a salt and/or an organic form. It has to pointed out that the efficacy of the use according to the invention is not necessarily directly and solely dependent on the resultant mineral concentration of the substrate : bioavailability has also to be taken into account. In another embodiment, said at least one mineral-rich or enriched yeast is a commercially available product, e.g. a product from the Danstar Ferment A.G. Mineral Enriched Yeast range.

Said yeast is advantageously rich in, or enriched in at least one mineral which is capable of altering the metabolism of an alcoholic fermentation.

A capacity of altering the metabolism of an alcoholic fermentation can be easily assessed by the person skilled in the art, e.g. by comparing the growth level of the fermentation micro-organism, and/or the rate of fermentation, and/or the secondary metabolites concentrations and/or the flavour profile, in the presence and in the absence of the mineral candidate under standard appropriate laboratory conditions. The word "mineral" herein also comprises oligoelements. Such a mineral is preferably a metal, and most preferably a divalent metal. It is advantageously chosen among the group consisting of zinc, magnesium, manganese. A most preferred mineral is generally zinc. But when it deals with negating the repressive effect of the calcium and thereby increasing sugar/alcohol conversion, magnesium is then preferred. Said at least one mineral-rich or enriched yeast can carry more than one nutrient mineral at a time, i.e. it can be a combination or a permutation of e.g. magnesium and zinc.

The use of said at least one mineral-rich or enriched yeast according to the invention is such that the mineral(s) contained therein or thereon is(are) released to the benefit of the fermentative micro-organism culture. Preferably, said at least one mineral-rich or enriched yeast contains, before being used, a concentration ranging from about 1.000 to about 200.000 ppm for each mineral it carries.

One of the many advantages of the process and use according to the invention lies in the fact that said at least one mineral-rich or enriched yeast can be supplied in any form appropriate to the precise fermentation process wherein it has to be used. It can be supplied in a living form, or in a dead form. It may be cellularly intact, but, as it is used as a nutrient source, and not for a cell production, it also can be cellularly slightly ruptured.

Said at least one mineral-rich or enriched yeast can indeed be used under a variety of forms which include a dry form, a liquid form, a frozen form, a freeze-dried form, a paste, or a powder. It may have been sterilised or not. It may be used on its own or as part of a mixture of other products.

The process and use according to the invention can thus be seen as the use of at least one sacrificial yeast as a nutrient source in alcoholic fermentations. Another advantage lies in the fact that said use can be performed, as desired, at any step of the fermentation process. A simple direct addition of said at least one mineral-rich or enriched yeast at at least one step of the fermentation process is efficient. It may thus be added directly into the boiling vessel, and/or the fermenter, and/or any vessel between the two, and/or into the fermentation micro-organism holding or propagating vcssels. For example, in beer production, the addition to the wort can be performed during alcohol production process or fermentation micro-organism propagation process, before or after boiling.

Said at least one mineral-rich or enriched yeast can thus be added directly to the wort so that it is killed during the wort boiling stage. It may be also added to the cooled wort prior to, during or after yeast pitching. Preferably said at least one mineral-rich or enriched yeast is added to the boiling wort. Advantageously, said use according to the invention is performed so that said yeast is used at such a quantity and/or at such a concentration in said mineral that it leads to an increase of at least about 0.05 ppm of the mineral content of the substrate of said fermentation . The fermented substrate itself may be distilled or not.

The use according to the invention is particularly efficient in that it accelerates alcoholic fermentation velocity greater than when the mineral concentration is raised by the addition of the equivalent concentration of mineral when derived from a mineral salt. A synergetic effect can moreover be outlined when comparing to the addition of mineral salt on one hand and dead yeast on the other hand (see *e*.*g*. laboratory tests 2 and 3 of example 1 for zinc). The fermentation duration needed therefore decreases (see examples below). The limit to primary fermentation is achieved faster, significantly in comparison to when the equivalent mineral concentration is derived from a mineral salt. The number of hours necessary to achieve the standard specific gravity (about 3.6°P or about 3.8°P for beer) is decreased: the time needed to achieve the attenuation degree of fermentation is decreased of several hours (about 20 hours in the below examples with zinc).

The use according to the invention also allows the fermentation to progress to absolute dryness, *i.e.* to an absence of residual fermentable sugars in the alcohol thus produced (see *e.g.* the below example 2). It not only allows a higher production of alcohol, but also a qualitatively better one (see *e.g.* the below example 1). And last, but not least, despite said fermentation acceleration, the alcohol produced according to the invention tastes equal to or better, in comparison to when the equivalent mineral concentration is derived from a mineral salt (see also the below example 3).

The process and use according to the present invention are of first interest for the beer industry, but it can also apply to any alcoholic fermentation beit cereal based, such as whisky or sake as well as fruit, sugar or honey based fermentations, such as wine, brandy, cider, fruit wines, mead, rhum, tequila, industrial alcohols, potable alcohols, vodka, gin, etc.

The present invention also relates to the use of at least one mineral-rich or mineral-enriched yeast as a nutrient source as herein described for the production of alcohol by a fermentation process.

Technical features and advantages of the present invention are herein further illustrated by several examples, which are given for illustration purposes and are in no way intented in restricting the scope of the invention. In these examples, reference is made to :
- figure 1 which represents the results of brewery trial 1 (Extracts [°Balling] as a function of the number of fermentation days), and to
- figure 2 which represents, in a similar way as for figure 1, the results of brewery trial 2.

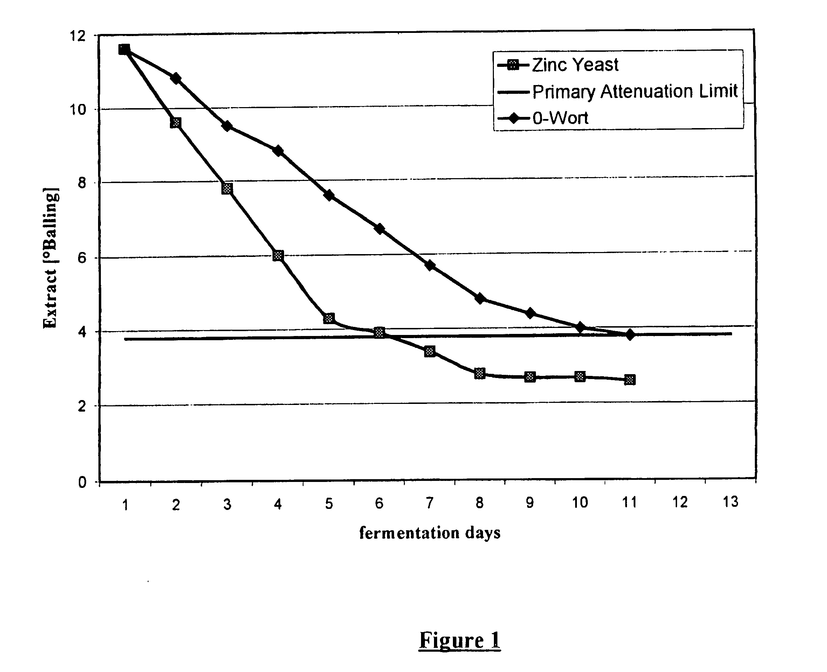

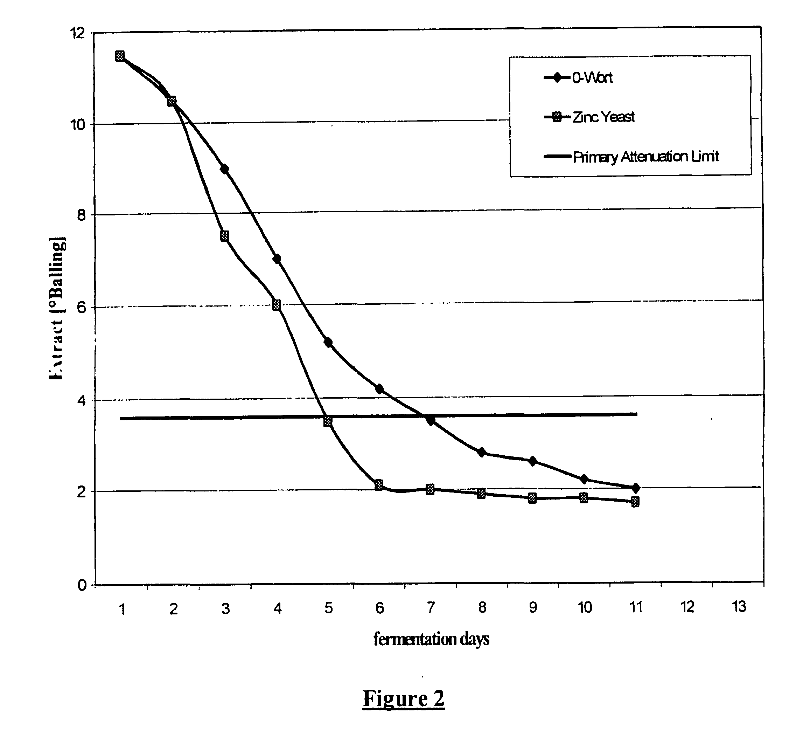

In figures 1 and 2, the legend is the following :
lozenges : O-wort
squares : zinc yeast
horizontal line : primary attenuation limit

### EXAMPLE 1: FERMENTATION SPEED

Three laboratory tests and two brewery trials were carried out to show the relative effectiveness of sacrificial zinc yeast and zinc chloride addition to worts containing different natural concentrations of zinc, and then fermented by yeasts containing different natural concentrations of zinc.

### Materials and Methods

### Materials

### Yeast strains and provenance (laboratory tests)

In the results herein reported, the yeast sources were as follows :
The yeast strain used in all laboratory trials was a strain of S. cerevisiae (lager type) obtained from four different commercial breweries in Germany. The sample taken was from their stock designated to be used for their next fermentation. The strain is for tests one, two and three, and in brewery trial 2 is deposited with the Technical University of Munich-Weihenstephan Hefebank, and designated as strain number W 34/70. The strain used in brewery trial 1 is deposited with the Technical University of Munich-Weihenstephan Hefebank, and designated as strain number W 120.
The yeast used for all laboratory trials was obtained, when needed, in the form of a cream from the appropriate brewery. The cream was centrifuged in a SORVAL® RC5B centrifuge at 2700g for 10 minutes and the supernatant was discarded. The yeast paste was weighed and resuspended in cooled wort, aerated and then added directly to the fresh worts. The zinc content of the yeast was measured before pitching.

### Media

### Laboratory Test Fermentations

The wort used was obtained from three commercial breweries in Germany. In test number one the wort is used to make their "helle" type beer. In test number two the wort is used to make their "festbier". In test number three the wort is used to produce "pilsner" type beer. In all cases the wort was collected at the end of the boil and was therefore hopped to the normal level of that product for that particular beer. The worts had not been treated in the respective brewery in any way to alter the natural level of zinc. The worts were boiled for fifteen minutes before being cooled to the fermentation temperature, 10°C, and pitched with fermentation yeast.

### Brewery Trials

Two brewery trials were carried out in two commercial breweries in Germany. The brewery in trial one was the Brauerei Kreiger, 944505 Landau, D. Isar, Bavaria, Germany. During a normal commercial production phase, two consecutive "helle" type worts, number 22 and 23, produced on 20^{th} and 21^{st} of April 1999 respectively, were designated for experimental observation. They were produced from the same recipe, one immediately after the other, from the same malt and hop stocks, and brewing water. 110 hectolitres of wort was collected from each brew. The brewery in trial two was, the Privatbrauerei Kitzmann, Kitzmann Bräu KG, Südliche Stadmauerstrasse 25, 91954 Erlangen Bavaria, Germany. During a normal commercial production phase, one wort, designated brew number 120, produced on the 26^{th} of April 1999, containing 286 litres of wort at 11.6° Balling, was separated into two fermenters each containing one hundred and forty-three hectolitres of "pilsner" wort. Both temperature profiles of the fermentations were as per normal for that brewery for that beer type.

### Zinc Preparation

### Laboratory tests

Mineral zinc, when used, was added in the form of the salt zinc chloride. This salt is used extensively by breweries throughout the World.
Zinc measurements on the wort and yeast samples were carried out by atomic absorption spectrometry as per the MEBAK® standard brewery analytical procedures, see *e.g* Lutz,A.: Bestrmmung, Vorkommen und Verhalten von Kontammationen durch verschiede umweltrelevante Spurenelemente in Bereich der Brauerei, Dissertation TU München, (1996), S21 ff.

### Preparation of Sacrificial zinc Yeast.

A sacrificial zinc yeast can be prepared by the person skilled in the art by any method convenient for producing a zinc-rich yeast. Standard methods use the incorporation, absorption and/or adsorption of zinc by the yeast. It should be noted that to implement the present invention, the yeast could be living or dead.

The preparations used in the trials herein reported were produced in some manner as per the following method.

Zinc, at a concentration of between 1,000 and 200,000 ppm (relative to the weight of the yeast or yeast fraction, as measured on a dry weight basis), in the form of zinc sulphate, chloride, acetate, phosphate, or some other appropnate zinc form is added to a live or dead culture of S. *cerevisae* at a temperature of about 4 to about 40° Celsius (preferably of about 25-32°C) at pH of between about 3.5 to about 7 (preferably about 4.6 to about 6.6), for a period of 1 to 20 or 24 hours so as to allow the culture to incorporate, absorb and/or adsorb the zinc.

Two base dry zinc yeast preparations were used in the trials;
Preparation one contained 10,500 ppm mineral zinc
Preparation two contained 70,000 ppm mineral zinc

Other zinc yeast preparations are also commercially available from Danstar Ferment AG, 20 Alpenstrasse, 6301 ZUG, Switzerland (MEY® Zn 50).

All the zinc preparations were added to the boiling worts, at the start of boiling.

In summary, the state of zinc in the yeasts and worts in the three laboratory tests and two brewery trials is as follows in :

**Table I**

| | Test 1 | Test 2 | Test 3 | Trial 1 | Trial 2 |
|---|---|---|---|---|---|
| Wort ppm | 0.2 | 0.06 | 0.1 | 0.06 | 0.1 |
| Fermentation Yeast mg/100g dry | 5.9 | 1.3 | 4.1 | 1.3 | 5 |

### Fermentation Yeast Preparation

A portion of the test yeast, 30 g of the paste, was re-suspended in 250 millilitre of boiled wort and aerated for approximately five minutes by way of a magnetic stirrer. The yeast preparation was then divided into seven equal aliquots and pitched into the appropriate test wort.

Brewery trial one and brewery trial two were conducted similarly.

The yeasts for brewery trial one, and for brewery trial two, were collected from previous fermentations stored as a cream, under conditions of refrigeration, and pitched as per the normal procedure for the brewery.

The yeast used for fermentation was pitched at a level of 1.6 litres of yeast cream per hectolitre of wort (this is normal yeast handling and pitching procedures for this brewery) for brewery trial one, and of 1.8 litres for brewery trial two.

The zinc content of the yeast was measured before pitching.

### Experimental Methods

### Laboratory Tests

The sample of the brewery wort obtained was divided up into 2.0 litre aliquots. For laboratory test 1, seven different types of aliquots were prepared and additions were made to each as follows :
- Type 0.: No addition
- Type 1.: 0.6mg of zinc chloride, which produced a measured increase of 0.28 mg mineral zinc per litre present in the wort.
- Type 2.: 40 mg of sacrificial zinc yeast preparation (at 10,500 ppm zinc) which produced a measured increase of 0.24 ppm zinc present in the wort.
- Type 3.: 160 mg of sacrificial zinc yeast preparation (at 10,500 ppm zinc) which produced a measured increase of 0.805 ppm zinc present in the wort.
- Type 4.: 8 mg of sacrificial zinc yeast preparation (at 70,000 ppm zinc) which produced a measured increase of 0.26 ppm zinc present in the wort.
- Type 5.: 16 mg of sacrificial zinc yeast preparation (at 70,000 ppm zinc) which corresponds to 0.88 ppm measured additional zinc present in the wort.

For laboratory test 2, six different types of aliquots were prepared and additions were made to each as follows :
- Type 0.: No addition
- Type 1.: 0.6 mg of sacrificial zinc yeast preparation (at 70,000 ppm zinc) which corresponds to 0.16 ppm measured additional zinc in the wort.
- Type 2.: 30 mg of sacrificial zinc yeast preparation (at 70,000 ppm zinc) which corresponds to 0.8 ppm measured additional zinc in the wort.
- Type 3.: 160 mg of dry dead brewers yeast, which corresponds to no measured increase in the zinc content of the wort.
- Type 4.: 0.6 mg of zinc chloride, which corresponds to 0.15 ppm measured additional zinc in the wort.
- Type 5.: Addition of 80 mg dry dead brewers yeast plus 0.6 mg zinc chloride which corresponds to 0.15 ppm measured additional zinc in the wort.

For laboratory test 3, six different types of aliquots were prepared and additions were made to each as follows :
- Type 0.: No addition
- Type 1.: 4.6 mg of sacrificial zinc yeast preparation (at 70.000 ppm zinc) which corresponds to 0.26ppm measured additional zinc in the wort.
- Type 2.: 160mg of dry dead brewers yeast, which corresponds to 0.01 ppm measured increase of zinc in the wort.
- Type 3.: 35 mg of sacrificial zinc yeast preparation (at 70,000 ppm zinc) which actually corresponds to a measured increase 1.12 ppm of zinc in the wort.
- Type 4.: 2.5 mg of zinc chloride, which actually corresponds to a measured increase 0.25 ppm measured increase of zinc in the wort.
- Type 5.: Addition of 160mg dry dead brewers yeast plus 0.6g zinc chloride, which corresponded to a measured increase of 0.29 ppm zinc in the wort.

Each aliquot was boiled for fifteen minutes. The zinc preparations were added at the start of the boil. The boiling vapours were condensed and returned to the respective lot in order to minimise evaporative loss. The wort preparations were sealed, allowed to cool to 8°C then, pitched with the appropriate quantity of yeast.

### Brewery Trials

For brewery trial 1, as for brewery trial 2, one fermenter received the equivalent of 0.30 ppm of additional zinc whilst the other received nothing.

### Fermentation

### Laboratory Tests one, two and three

Fermentation was carried out in a constant temperature room at approximately 10°C until a density of 3.6°P had been achieved. This is normal for beers that arc transferred to lager under conditions of refrigeration so that secondary fermentation and maturation can take place.

### Brewery Trials

Fermentation was carried under the standard temperature programme for that particular wort type. The standard and test worts were subject to the same profile.

### Measurements

### Laboratory tests one, two and three.

Samples were drawn from the wort prior to pitching with yeast and the zinc content was measured.

Fermentation progress was measured by a standard brewing densitometer and recorded in degrees Plato after compensation for temperature effects.

At the beginning of fermentation zinc determinations were carried out on the untreated and treated worts and the pitching yeast. Determinations had previously been carried out on the zinc yeast preparations.

The finished beer in all three trials was analysed using a SCABA® "automatic beeranalyser" from PERSTOP ANALYTICAL, SWEDEN, for alcohol concentration as expresses in volume per volume.

### Brewery trials

During fermentation samples were drawn at regular intervals, and progress of the fermentation, was measured by the drop in density of the wort, as expressed in degree Balling.

### Results and Conclusions

### Laboratory Tests 1, 2, and 3

The time taken, for each fermentation to achieve 3.6 degree Plato, which is judged to be when primary fermentation is complete and the alcohol concentrations after two hundred and forty hours of fermentation, are detailed below in table 2 for laboratory test 1, in table 3 for laboratory test 2, and in table 4 for laboratory test 3.

**Table 2**

| In Addition | | Zinc Chloride | Zinc Yeast | Zinc Yeast | Zinc Yeast | Zinc Yeast |
|---|---|---|---|---|---|---|
| Zinc Conc. ppm | 0.2 | 0.48 | 0.44 | 1.05 | 0.46 | 1.08 |
| Hours to achieve 3.6°P | 186 | 168 | 150 | 127 | 110 | 110 |
| Hours difference from standard | 0 | -14 | -36 | -59 | -76 | -76 |
| % of standard fermentation time | 100 | 90 | 81 | 68 | 59 | 59 |
| Alcohol by Volume | 4.74 | 4.92 | 5.05 | 5.3 | 5.26 | 5.3 |

**Table 3**

| | **0** | **1** | **2** | **3** | **4** | **5** |
|---|---|---|---|---|---|---|
| Addition | | Zinc Yeast | Zinc Yeast | Dead yeast | Zinc Chloride | Dead Yeast + zinc chloride |
| Zinc Conc. ppm | 0.06 | 0.22 | 0.86 | 0.06 | 0.21 | 0.21 |
| Hours to achieve 3.6°P | 175 | 153 | 148 | 186 | 156 | 163 |
| Hours difference from standard | 0 | -20 | -24 | +10 | -14 | -2 |
| % of standard fermentation time | 100 | 87 | 84 | 106 | 89 | 93 |
| Alcohol by Volume | 4.86 | 5.02 | 5.05 | 4.93 | 4.91 | 4.96 |

**Table 4**

| | 0 | 1 | 2 | 3 | 4 | 5 |
|---|---|---|---|---|---|---|
| Addition | | Zinc Yeast | Dead Yeast | Zinc Yeast | Zinc Chloride | Dead Yeast + Zinc chloride |
| Zinc Conc. ppm | 0.1 | 0.36 | 0.11 | 1.22 | 0.35 | 0.39 |
| Hours to achieve 3.6°P | 182 | 162 | 180 | 158 | 177 | 168 |
| Hours difference from standard | 0 | -20 | -2 | -24 | -5 | -14 |
| % of standard fermentation time | 100 | 89 | 99 | 87 | 97 | 92 |
| Alcohol by Volume | 5.78 | 5.86 | 5.81 | 5.86 | 5.6 | 5.74 |

### Conclusions

1. In all fermentation tests where sacrificial zinc yeast was added, fermentation speed was improved in comparison to the standard wort, wort with added zinc chloride, and, when tested, wort with added dead yeast, and wort with added zinc chloride plus dead yeast.
2. In all fermentation tests where sacrificial zinc yeast was added, the standard specific gravity, designated for onward processing of the beer for lagering, was achieved faster than the standard wort, wort with added zinc chloride, and, when tested, wort with added dead yeast, and wort with added zinc chloride plus dead yeast. The time to achieve this degree of fermentation attenuation was at least twenty hours and as great as seventy-six hours less than the standard.
3. Against the test where zinc chloride was added, the sacrificial zinc yeast trials achieved the standard fermentation attenuation at least six and as great as sixty-two hours sooner.
4. Where the zinc addition was at a similar level from mineral zinc (zinc chloride), and biological zinc (sacrificial zinc yeast), the sacrificial zinc yeast experiments were measurably and significantly faster.
5. In all fermentation tests where sacrificial zinc was added, the final concentration of alcohol produced after ten days, was greater than the standard wort, the wort containing zinc chloride, and when tested, the wort containing inactivated yeast and the wort containing zinc chloride plus inactivated yeast.

### Brewery trial 1 and trial 2

Data collected from the brewery fermentation trial number 1 and 2 are displayed in the below table 5. Graphical representation of these data are displayed in figure 1 for brewery trial 1, and in figure 2 for brewery trial .

**Table 5**

| | Trial 1 (primary attenuation limit 3.8) | | Trial 2 (primary attenuation limit 3.6) | |
|---|---|---|---|---|
| Fermentation days | 0-Wort | Zinc Yeast | 0-Wort | Zinc Yeast |
| 0 | 11.6 | 11.6 | 11.5 | 11.5 |
| 1 | 10.8 | 9.6 | 10.5 | 10.5 |
| 2 | 9.5 | 7.8 | 9 | 7.5 |
| 3 | 8.8 | 6 | 7 | 6 |
| 4 | 7.6 | 4.3 | 5.2 | 3.5 |
| 5 | 6.7 | 3.9 | 4.2 | 2.1 |
| 6 | 5.7 | 3.4 | 3.5 | 2 |
| 7 | 4.8 | 2.8 | 2.8 | 1.9 |
| 8 | 4.4 | 2.7 | 2.6 | 1.8 |
| 9 | 4 | 2.7 | 2.2 | 1.8 |

### Conclusions

1. The fermentation containing sacrificial zinc yeast was faster than the standard untreated wort.
2. In the fermentation trials where sacrificial zinc yeast was added, the standard specific gravity, 3.8 and 3.6 degrees Balling respectively, designated for onward processing of the beer for lagering was achieved one hundred and forty hours sooner (trial 1), and forty eight hours sooner (trial 2), than the standard untreated wort.

The two beers produced were tasted, by a party of professional brewers and others with professional expertise in beer tasting. The trial beer was judged to be at least as good as the standard and was preferred by many.

The tasters noted that both test beers were particularly lower in "sulphitic" character. This is of particular importance and significance for not only does it indicate a "cleaner" beer it indicates a possibility of advancing the maturation process and thus reducing lager times and costs. At the cellular level, zinc addition according to the invention can show the following stimulatory effects: stabilising proteins and membrane systems, acting as a catalytic centre of essential enzymes (e.g. alcohol dehydrogenase, aldolase and acetaldehyde dehydrogenase), enhancing riboflavin synthesis, activating acid and alkaline synthesis, stimulating the uptake of maltose and maltotriose.

### EXAMPLE 2 : Residual sugar analysis

After two hundred and forty hours of fermentation of laboratory test the residual sugars in all the beers of test 1 were analysed by gas liquid chromatography. The results are detailed in the below table 6.

**Table 6**

| **Experiment** | **0** | **1** | **2** | **3** | **4** | **5** |
|---|---|---|---|---|---|---|
| Sugars as mg/100ml | | Zinc Chloride | Zinc Yeast | Zinc Yeast | Zinc Yeast | Zinc Yeast |
| Glucose | 0 | 0 | 0 | 0 | 0 | 0 |
| Fructose | 0 | 0 | 0 | 0 | 0 | 0 |
| Sucrose | 0 | 0 | 0 | 0 | 0 | 0 |
| Maltose | 0.69 | 0.47 | 0 | 0 | 0 | 0 |
| Maltotriose | 0.15 | 0.09 | 0 | 0 | 0 | 0 |
| Total | 0.84 | 0.56 | 0 | 0 | 0 | 0 |

### Conclusions

It is clear from the data presented above that the inclusion of sacrificial zinc yeast permits the fermentation to progress to absolute dryness. That is to say there are no fermentable sugars left in the beer. This is highly significant as it will permit brewers to produce beers free of residual fermentable sugars much quicker that at present. The technical features of the process according to the invention thus lead to more alcohol produced, thereby giving an economic advantage.

### EXAMPLE 3 : Quality and quantity of secondary flavour compounds.

After the completion of the fermentations in laboratory test 2 (see the above example 1) the beer was subject G.L.C. analysis to see if the accelerated fermentations affected the quality and quantity of secondary flavour compounds. The results are detailed in the below table 7.

**Table 7**

| Mg/L | **0** | **1** | **2** | **3** | **4** | **5** |
|---|---|---|---|---|---|---|
| | 0 | Zinc Yeast | Zinc Yeast | Dead Yeast | Zinc chloride | Dead yeast + Zinc chloride |
| Diacetyl | 0.22 | 0.27 | 0.29 | 0.22 | 0.24 | 0.24 |
| Pantadione 2.3 | 0.21 | 0.25 | 0.26 | 0.22 | 0.22 | 0.23 |
| Acetadehyde | 24.2 | 25.6 | 27.9 | 25.9 | 24.2 | 25.5 |
| Ethyl acetate | 28.7 | 25.2 | 30.9 | 27.3 | 28.7 | 27.8 |
| i-Butanol | 7.6 | 7.5 | 8.1 | 7.4 | 7.6 | 8 |
| n-Propanol | 10.2 | 10.4 | 11.4 | 10.7 | 10.4 | 11.2 |
| Amyl acetate | 2.8 | 2.5 | 3.2 | 2.7 | 2.9 | 2.8 |
| Amyl alcohol | 52.1 | 49.3 | 52.7 | 50.9 | 51.4 | 52 |

### Conclusions

It is evident from the results above that accelerating the fermentation, by way of sacrificial zinc yeast, has no significant negative effect on the major, secondary, organoleptically active metabolites. Such a negative effect has indeed not been observed in any of the tests and trials performed (see the above example 1). This is particulary striking effect of the process of the invention which allows an accelerated fermentation without negatively affecting the alcohol profile of the product. These conclusions on the quality of the beer produced according to the invention were further confirmed by blind taste panels.
This is a significant finding as it allows a standard beer to be produced at a faster, and therefore cheaper rate.

It will be apparent to those skilled in the art that the process of the present invention which comprises the use of a mineral-rich yeast, and in particular of a zinc-rich yeast, as a fermentation nutrient is a very valuable technical contribution to additive-free brewing. It will also be apparent that the foregoing examples have been for purposes of illustration, and that a number of changes and modifications can be made without departing from the scope of the invention. The present invention illustrated with a zinc-rich yeast can thus be implemented without undue burden with a yeast rich in any mineral or combination of minerals appropriate to a yeast growth enhancement process, *e.g.* magnesium, manganese.

## Claims

1. Process for alcoholic fermentation comprising the use of a fermentation micro-organism, **characterized in that** it also comprises the use of at least one mineral-rich or mineral-enriched yeast as a nutrient source for said fermentation.

2. Process according to claim 1, **characterized in that** said at least one mineral-rich or enriched yeast belongs to the *Saccharomyces* genera or to the *Kluyveromyces* genera.

3. Process according to any one of claim 1 or 2, **characterized in that** said at least one mineral-rich or mineral-enriched yeast is prior to use obtainable by adding 1,000 to 200,000 ppm (relative to the weight of the yeast, as measured on a dry weight basis) of a salt of said mineral to a live culture of said micro-organism at a temperature of 4-40°C at a pH of between 3.5 to 7.0, for a period of 1-24 hours, so as to allow said micro-organism to incorporate said mineral.

4. Process according to claim 3, **characterized in that** said temperature is in the 25-32°C range.

5. Process according to claim 3, **characterized in that** said pH is in the 4.6-6.6 range.

6. Process according to claim 3, **characterized in that** said time period is in the 2-16 hour range.

7. Process according to any one of claims 3-6, **characterized in that** said salt is chosen among the group consisting of acetate, caprylate, carbonate, chloride, chromate, gluconate, iodate, lactate, oleate, oxide, perchlorate, peroxide, phosphate, salicylate, sulphate, sulphide, tartarate or valerate.

8. Process according to any one of claims 3-7, **characterized in that** said mineral incorporation corresponds to an absorption and/or an adsorption.

9. Process according to any one of claims 1 to 8, **characterized in that** said mineral is a metal capable of altering the metabolism of said fermentation.

10. Process according to any one of claims 1 to 9, **characterized in that** said mineral is chosen among the group consisting of zinc, magnesium and manganese.

11. Process according to any one of the preceding claims, **characterized in that** said at least one mineral-rich or enriched yeast contains before being used a concentration in said mineral ranging from 1,000 to 200,000 ppm .

12. Process according to any one of the preceding claims, **characterized in that** said at least one mineral-rich or enriched yeast is used under a form chosen among the group consisting of a living form and a dead form.

13. Process according to any one of the preceding claims, **characterized in that** said at least one mineral-rich or enriched yeast is used under a form chosen among the group consisting of a dry form, a liquid form, a frozen form, a freeze-dried form, a paste, a powder.

14. Process according to any one of the preceding claims, **characterized in that** said at least one mineral-rich or enriched yeast is used by directly adding it at at least one step of said fermentation process.

15. Process according to claim 14, **characterized in that** said addition is performed directly into at least one element selected from the group consisting of a fermenter, a boiling vessel, any vessel between the two, a fermentation micro-organism holding vessel, a fermentation micro-organism propagating vessel.

16. Process according to any one of the preceding claims, **characterized in that** said yeast is used at such a quantity and/or at such a concentration in said mineral that it leads to an increase of at least 0.05 ppm of the mineral content of the substrate of said fermentation.

17. Process according to any one of the preceding claims, **characterized in that** said alcoholic fermentation can lead to the production of beer.

18. Process according to any one of the preceding claims, **characterized in that** said alcoholic fermentation can lead to the production of an alcohol chosen among the group consisting of whisky or sake as well as fruit, sugar or honey based fermentations, such as wine, brandy, cider, fruit wines, mead, rhum, tequila, industrial alcohols, potable alcohols.

19. Use of a fermentation micro-organism and of at least one mineral-rich or mineral-enriched yeast as a nutrient source in the production of an alcohol by fermentation.

## Patentansprüche

1. Verfahren zur alkoholischen Fermentation, das die Verwendung eines Fermentationsmikroorganismus umfasst, **dadurch gekennzeichnet, dass** es auch die Verwendung wenigstens einer mineralreichen oder mineralangereicherten Hefe als Nährstoffquelle für die Fermentation umfasst.

2. Verfahren gemäß Anspruch 1, **dadurch gekennzeichnet, dass** die wenigstens eine mineralreiche oder mineralangereicherte Hefe zur Gattung *Saccharomyces* oder zur Gattung *Kluyveromyces* gehört.

3. Verfahren gemäß einem der Ansprüche 1 oder 2, **dadurch gekennzeichnet, dass** die wenigstens eine mineralreiche oder mineralangereicherte Hefe vor ihrer Verwendung erhältlich ist durch Zugabe von 1000 bis 200 000 ppm (relativ zum Gewicht der Hefe, gemessen auf Trockengewichtbasis) eines Salzes des Mineralstoffs zu einer Lebendkultur des Mikroorganismus bei einer Temperatur von 4-40 °C bei einem pH-Wert zwischen 3,5 und 7,0 während 1-24 Stunden, so dass der Mikroorganismus das Mineral aufnehmen kann.

4. Verfahren gemäß Anspruch 3, **dadurch gekennzeichnet, dass** die Temperatur im Bereich von 25 bis 32 °C liegt.

5. Verfahren gemäß Anspruch 3, **dadurch gekennzeichnet, dass** der pH-Wert im Bereich von 4,6 bis 6,6 liegt.

6. Verfahren gemäß Anspruch 3, **dadurch gekennzeichnet, dass** die Zeitdauer im Bereich von 2-16 Stunden liegt.

7. Verfahren gemäß einem der Ansprüche 3 bis 6, **dadurch gekennzeichnet, dass** das Salz aus der Gruppe ausgewählt ist, die aus Acetat, Caprylat, Carbonat, Chlorid, Chromat, Gluconat, Iodat, Lactat, Oleat, Oxid, Perchlorat, Peroxid, Phosphat, Salicylat, Sulfat, Sulfid, Tartrat oder Valerat besteht.

8. Verfahren gemäß einem der Ansprüche 3 bis 7, **dadurch gekennzeichnet, dass** die Mineralaufnahme einer Absorption (Resorption) und/oder einer Adsorption entspricht.

9. Verfahren gemäß einem der Ansprüche 1 bis 8, **dadurch gekennzeichnet, dass** der Mineralstoff ein Metall ist, das den Stoffwechsel der Fermentation ändern kann.

10. Verfahren gemäß einem der Ansprüche 1 bis 9, **dadurch gekennzeichnet, dass** der Mineralstoff aus der Gruppe ausgewählt ist, die aus Zink, Magnesium und Mangan besteht.

11. Verfahren gemäß einem der vorstehenden Ansprüche, **dadurch gekennzeichnet, dass** die wenigstens eine mineralreiche oder -angereicherte Hefe vor ihrer Verwendung eine Konzentration des Minerals im Bereich von 1000 bis 200 000 ppm enthält.

12. Verfahren gemäß einem der vorstehenden Ansprüche, **dadurch gekennzeichnet, dass** die wenigstens eine mineralreiche oder -angereicherte Hefe in einer Form verwendet wird, die aus der Gruppe ausgewählt ist, die aus einer lebenden Form und einer toten Form besteht.

13. Verfahren gemäß einem der vorstehenden Ansprüche, **dadurch gekennzeichnet, dass** die wenigstens eine mineralreiche oder -angereicherte Hefe in einer Form verwendet wird, die aus der Gruppe ausgewählt ist, die aus einer trockenen Form, einer flüssigen Form, einer gefrorenen Form, einer gefriergetrockneten Form, einer Paste und einem Pulver besteht.

14. Verfahren gemäß einem der vorstehenden Ansprüche, **dadurch gekennzeichnet, dass** die wenigstens eine mineralreiche oder -angereicherte Hefe verwendet wird, indem man sie in wenigstens einem Schritt des Fermentationsverfahrens direkt hinzufügt.

15. Verfahren gemäß Anspruch 14, **dadurch gekennzeichnet, dass** die Zugabe direkt in wenigstens ein Element erfolgt, das aus der Gruppe ausgewählt ist, die aus einem Fermenter, einem Siedegefäß, irgendeinem Gefäß zwischen den beiden, einem Fermentationsmikroorganismus-Vorratsgefäß und einem Fermentationsmikroorganismus-Vermehrungsgefäß besteht.

16. Verfahren gemäß einem der vorstehenden Ansprüche, **dadurch gekennzeichnet, dass** die Hefe mit einer solchen Menge und/oder mit einer solchen Konzentration des Mineralstoffs verwendet wird, dass sie zu einer Erhöhung des Mineralgehalts des Substrats der Fermentation von wenigstens 0,05 ppm führt.

17. Verfahren gemäß einem der vorstehenden Ansprüche, **dadurch gekennzeichnet, dass** die alkoholische Fermentation zur Produktion von Bier führen kann.

18. Verfahren gemäß einem der vorstehenden Ansprüche, **dadurch gekennzeichnet, dass** die alkoholische Fermentation zur Produktion eines alkoholischen Getränks führen kann, das aus der Gruppe ausgewählt ist, die aus Whisky oder Sake sowie Fermentationen auf der Grundlage von Früchten, Zucker oder Honig, wie Wein, Weinbrand, Apfelwein, Fruchtweinen, Met, Rum, Tequila, Industriealkoholen und Trinkalkoholen besteht.

19. Verwendung eines Fermentationsmikroorganismus und wenigstens einer mineralreichen oder mineralangereicherten Hefe als Nährstoffquelle bei der Produktion eines alkoholischen Getränks durch Fermentation.

## Revendications

1. Procédé de fermentation alcoolique comprenant l'utilisation d'un microorganisme de fermentation, **caractérisé en ce qu'**il comprend également l'utilisation d'au moins une levure riche en minéral ou enrichie en minéral en tant que source de nutriments pour ladite fermentation.

2. Procédé selon la revendication 1, **caractérisé en ce que** ladite au moins une levure riche en minéral ou enrichie en minéral appartient au genre *Saccharomyces* ou au genre *Kluyveromyces*.

3. Procédé selon l'une quelconque des revendications 1 ou 2, **caractérisé en ce que** ladite au moins une levure riche en minéral ou enrichie en minéral peut être obtenue, avant utilisation, en ajoutant 1 000 à 200 000 ppm (par rapport au poids de levure, mesuré sur la base du poids sec) d'un sel dudit minéral à une culture vivante dudit microorganisme, à une température allant de 4 à 40°C, à un pH compris entre 3,5 et 7,0 et pendant une durée de 1 à 24 heures, pour permettre audit microorganisme d'incorporer ledit minéral.

4. Procédé selon la revendication 3, **caractérisé en ce que** ladite température est dans la gamme allant de 25 à 32°C.

5. Procédé selon la revendication 3, **caractérisé en ce que** ledit pH est dans la gamme allant de 4,6 à 6,6.

6. Procédé selon la revendication 3, **caractérisé en ce que** ladite durée est dans la gamme allant de 2 à 16 heures.

7. Procédé selon l'une quelconque des revendications 3 à 6, **caractérisé en ce que** ledit sel est choisi dans le groupe constitué par l'acétate, le caprylate, le carbonate, le chlorure, le chromate, le gluconate, l'iodate, le lactate, l'oléate, l'oxyde, le perchlorate, le peroxyde, le phosphate, le salicylate, le sulfate, le sulfure, le tartrate ou le valérate.

8. Procédé selon l'une quelconque des revendications 3 à 7, **caractérisé en ce que** ladite incorporation du minéral correspond à une absorption et/ou à une adsorption.

9. Procédé selon l'une quelconque des revendications 1 à 8, **caractérisé en ce que** ledit minéral est un métal capable de modifier le métabolisme de ladite fermentation.

10. Procédé selon l'une quelconque des revendications 1 à 9, **caractérisé en ce que** ledit minéral est choisi dans le groupe constitué par le zinc, le magnésium et le manganèse.

11. Procédé selon l'une quelconque des revendications précédentes, **caractérisé en ce que** ladite au moins une levure riche en minéral ou enrichie en minéral contient, avant utilisation, une concentration en ledit minéral allant de 1 000 à 200 000 ppm.

12. Procédé selon l'une quelconque des revendications précédentes, **caractérisé en ce que** ladite au moins une levure riche en minéral ou enrichie en minéral est utilisée sous une forme choisie dans le groupe constitué par une forme vivante et une forme morte.

13. Procédé selon l'une quelconque des revendications précédentes, **caractérisé en ce que** ladite au moins une levure riche en minéral ou enrichie en minéral est utilisée sous une forme choisie dans le groupe constitué par une forme sèche, une forme liquide, une forme congelée, une forme lyophilisée, une pâte et une poudre.

14. Procédé selon l'une quelconque des revendications précédentes, **caractérisé en ce que** ladite au moins une levure riche en minéral ou enrichie en minéral est utilisée en l'ajoutant directement à au moins une étape dudit procédé de fermentation.

15. Procédé selon la revendication 14, **caractérisé en ce que** ladite addition est réalisée directement dans au moins un élément choisi dans le groupe constitué par une cuve de fermentation, un récipient d'ébullition, tout récipient entre les deux, un récipient de conservation de microorganisme de fermentation et un récipient de propagation de microorganisme de fermentation.

16. Procédé selon l'une quelconque des revendications précédentes, **caractérisé en ce que** ladite levure est utilisée en une quantité telle et/ou en une concentration telle dans ledit minéral qu'elle entraîne une augmentation d'au moins 0,05 ppm de la teneur en minéral du substrat de ladite fermentation.

17. Procédé selon l'une quelconque des revendications précédentes, **caractérisé en ce que** ladite fermentation alcoolique peut mener à la production de bière.

18. Procédé selon l'une quelconque des revendications précédentes, **caractérisé en ce que** ladite fermentation alcoolique peut mener à la production d'un alcool choisi dans le groupe constitué par le whisky ou le saké, ainsi que des fermentations à base de fruit, de sucre ou de miel, tels le vin, l'eau de vie, le cidre, les vins de fruit, l'hydromel, le rhum, la tequila, les alcools industriels et les alcools de bouche.

19. Utilisation d'un microorganisme de fermentation et d'au moins une levure riche en minéral ou enrichie en minéral en tant que source de nutriments pour la production d'un alcool par fermentation.
